(19)

Europäisches
Patentamt
European
Patent Office
Office européen
des brevets

(11)  EP 3 315 957 A1

(12)  **EUROPEAN PATENT APPLICATION**
published in accordance with Art. 153(4) EPC

(43)  Date of publication:
02.05.2018  Bulletin 2018/18

(51)  Int Cl.:
*G01N 27/327* (2006.01)     *G01N 33/53* (2006.01)

(21)  Application number: 16818159.2

(22)  Date of filing: 22.06.2016

(86)  International application number:
PCT/KR2016/006620

(87)  International publication number:
WO 2017/003126 (05.01.2017 Gazette 2017/01)

(84)  Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30)  Priority:  **29.06.2015  KR 20150092111**

(71)  Applicant: **SNU R&DB Foundation
Seoul 08826 (KR)**

(72)  Inventors:
• **PARK, Young June
Seoul 08807 (KR)**
• **CHOI, Seong Wook
Seoul 08790 (KR)**

(74)  Representative: **Boden, Keith McMurray
Fry Heath & Spence LLP
Unit A, Faraday Court
Faraday Road
Crawley, West Sussex RH10 9PU (GB)**

(54)  **BIO-SENSOR AND BIO-SENSOR ARRAY**

(57)  A bio-sensor according to embodiments of the present invention is a bio-sensor configured to detect a specific target, the bio-sensor includes a substrate, a first electrode and a second electrode disposed on the substrate and not electrically connected to each other, and probes disposed on the substrate, the first electrode, and the second electrode and coupled to the target.

[FIG. 2]

# Description

[Technical Field]

The present invention relates to a bio-sensor and a bio-sensor array.

[Background Art]

A bio-sensor according to a related art uses three electrodes. The three electrodes are referred to as a working electrode, a reference electrode, and a counter electrode. The presence of a target and/or a concentration of the target is detected by generating a voltage between the working electrode and the reference electrode to provide a target voltage and detecting a current value obtained from the working electrode and the counter electrode.

[Disclosure]

[Technical Problem]

In a case in which the presence of a target object and/or a concentration of the target object included in an electrolyte solution should be detected by a bio-sensor according to a related art, a voltage should be applied to any one of a working electrode and a reference electrode in a state in which a probe is immersed in the electrolyte solution.

In the case in which the voltage is applied through the probe, since a voltage drop (IR drop) occurs as the electrolyte solution is away from the probe due to electrical resistance of the electrolyte solution, it is difficult to apply a target voltage thereto. Since the application of the target voltage is unclear, it is not clear whether a detection result is due to an instrument error or the voltage drop, and thus it is difficult to ensure accuracy of the detection result.

Furthermore, a probe material selectively coupled to a target to be detected is formed, patterned, and selectively disposed on a conventional bio-sensor. Accordingly, accuracy of detection is reduced due to a change in a physical property of the probe material during a patterning process.

The present embodiment is for solving the above-described problems of the related art, and one main purpose thereof is to provide a bio-sensor capable of applying an accurate voltage using two electrodes. In addition, another main purpose thereof is to provide a bio-sensor in which detection accuracy is not reduced because a process in which a probe material is selectively disposed thereon is not performed.

[Technical Solution]

One aspect of the present invention provides a bio-sensor configured to detect a target, the bio-sensor including a substrate, a first electrode and a second electrode disposed on the substrate and not electrically connected to each other, and probes disposed on the substrate, the first electrode, and the second electrode and coupled to the target.

Another aspect of the present invention provides a bio-sensor configured to detect a target which is a biomaterial, the bio-sensor including a substrate, a first electrode and a second electrode disposed on the substrate, not electrically connected to each other, and having different surface areas, and probes disposed on the substrate, a detection electrode, and a common electrode and coupled to the target.

Another aspect of the present invention provides a bio-sensor array in which bio-sensors configured to detect a target, which is a biomaterial, are disposed, the bio-sensor array including a substrate, a plurality of island electrodes disposed on the substrate, a common electrode configured to surround the plurality of island electrodes disposed on the substrate and not in electrical contact with the plurality of island electrodes, and probes randomly disposed on the substrate, the plurality of island electrodes, and the common electrode and specifically coupled to the target, wherein surface areas of the plurality of island electrodes are smaller than that of the common electrode.

[Advantageous Effects]

According to the present embodiment, since a first electrode and a second electrode with different surface areas are used, an accurate voltage can be applied to the electrodes even though three electrodes are not used. Therefore, there is an advantage in that a voltage which is more accurate than that of a related art is economically generated and a target material can be detected.

Furthermore, since a voltage between an electrode and a solution can be accurately adjusted according to the present embodiment, there is an advantage in that a change in faradaic current can be accurately detected.

Since a probe is not patterned according to the present embodiment, there is no worry that physical properties of the probe will be changed. Therefore, there is an advantage in that a target material can be more accurately detected in comparison to a related art.

[Description of Drawings]

FIG. 1 is a top view illustrating a bio-sensor according to an embodiment of the present invention.
FIG. 2 is a schematic cross-sectional view illustrating the bio-sensor according to the embodiment.
FIG. 3A is a schematic view illustrating an electrical double layer formed between first and second electrodes and an electrolyte, and FIG. 3B is a view illustrating an example of a state in which the electrical double layer is formed from an electrical viewpoint.

FIG. 4 is a view illustrating an example of a state in which targets (T) are coupled to probes (P).

FIG. 5 is a schematic view illustrating the bio-sensor according to the present embodiment formed in an array type.

[Modes of the Invention]

Since the description for the present invention only discloses embodiments for structurally and functionally describing the present invention, a scope of the present invention is not to be interpreted as being limited to the embodiments described in the specification. That is, since the embodiments may be variously modified and have various forms, it should be understood that the scope of the present invention includes various equivalents that may realize the spirit of the present invention.

Meanwhile, terms used in the present invention should be interpreted as follows.

Since the terms "first," "second," and the like are used herein to distinguish one element from another, the scope of the present invention is not to be limited by the terms. For example, a first element could be termed a second element, and a second element could be termed a first element.

Elements of the invention referred to as singular may number one or more unless the context clearly indicates otherwise. It should be further understood that the terms "comprise," "comprising," "include," or "including," when used herein, specify the presence of stated features, numbers, steps, operations, elements, components, or groups thereof, but do not preclude the presence or addition of one or more other features, numbers, steps, operations, elements, components, or groups thereof.

The expression "and/or," when describing the embodiments of the present invention, is used to refer all or one of the related items. For example, the expression "A and/or B" should be understood as "A," and "B," and "A and B."

Unless otherwise defined, all the terms used herein have the same meaning as commonly understood by one of ordinarily skill in the art to which this invention belongs. It should be further understood that terms, such as those defined in commonly used dictionaries, should be interpreted as having a meaning that is consistent with their meaning in the context of the relevant art, and are not to be interpreted in an idealized or overly formal sense unless expressly so defined herein.

Sizes, heights, thicknesses, and the like in the drawings referred to when describing the embodiment of the present invention are intentionally exaggerated and expressed for convenience of description and to facilitate understanding, and are not enlarged or shrunk according to a ratio. In addition, some elements illustrated in the drawings may be intentionally expressed as being smaller, and the other elements may be intentionally expressed as being larger.

The expression "A and B are coupled" is used in the present specification to refer to a case in which A and B are physically coupled in a state in which chemical structures thereof are maintained, and also refers to a case in which A and B react and physical or chemical structures thereof are changed.

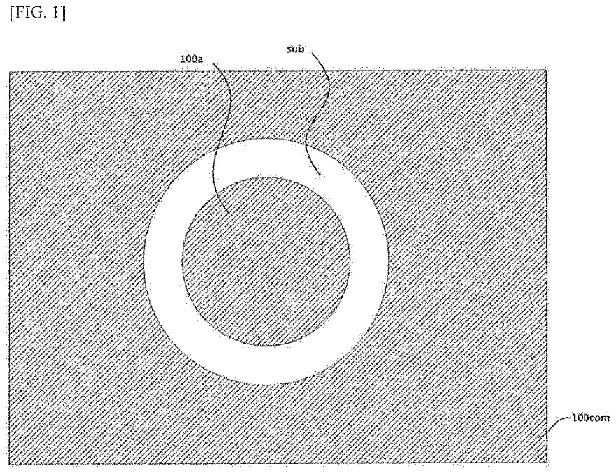

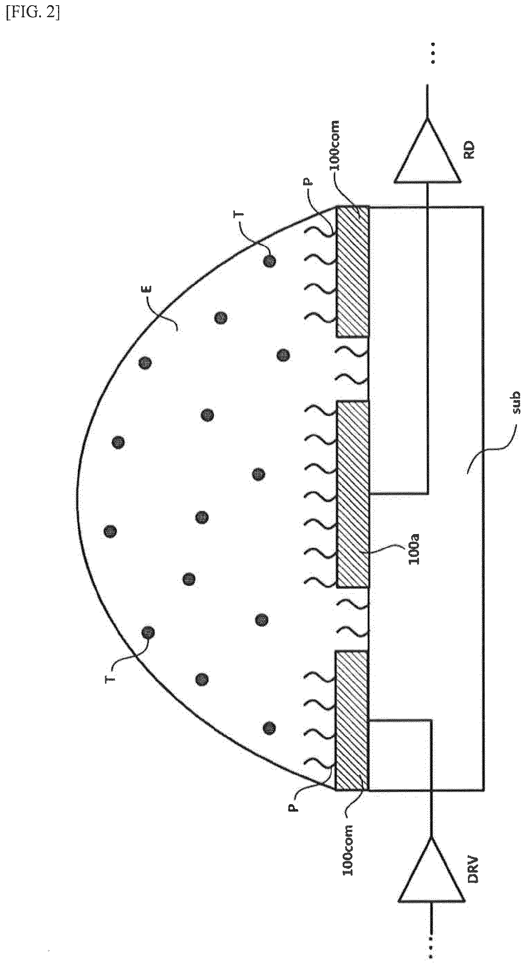

Hereinafter, a bio-sensor according to the present embodiment will be described with reference to the accompanying drawings. FIG. 1 is a top view illustrating a bio-sensor according to an embodiment of the present invention, and FIG. 2 is a schematic cross-sectional view illustrating the bio-sensor according to the embodiment. Referring to FIGS. 1 and 2, the bio-sensor according to the embodiment is a bio-sensor for detecting a specific target, and the bio-sensor includes a substrate, a first electrode and a second electrode disposed on the substrate and not electrically connected to each other, and probes disposed on the substrate, the first electrode, and the second electrode to be coupled to a target.

The bio-sensor according to the present embodiment is the bio-sensor for detecting the specific target, and the bio-sensor includes the substrate, the first electrode and the second electrode disposed on the substrate, not electrically connected to each other, and having different surface areas, and the probes disposed on the substrate, a detection electrode, and a common electrode to be coupled to the target.

A first electrode 100a and a second electrode 100com are located on one surface of a substrate sub. In addition, probes P may be located on the one surface of the substrate sub. The substrate comes into contact with an electrolyte solution E including targets T. Accordingly, the substrate is formed of a material which does not electrochemically react with the electrolyte solution E even when in contact with the electrolyte solution E. For example, the substrate sub is formed of glass. As another example, the bio-sensor may be formed through a semiconductor process, and according to the present embodiment, the substrate may be a silicon substrate.

The first electrode 100a is located the one surface of the substrate and is not electrically connected to the second electrode 100com. The second electrode 100com is located on the one surface of the substrate like the first electrode 100a. A surface area of the first electrode 100a configured to come into contact with the electrolyte is different from that of the second electrode 100com configured to come into contact with the electrolyte. For example, the surface area of the second electrode 100com configured to come into contact with the electrolyte solution E may be ten times or more greater than that of the first electrode 100a configured to come into contact with the electrolyte solution E.

The first electrode 100a and the second electrode 100com come into contact with the electrolyte solution E and apply a voltage to the electrolyte solution E.

Accordingly, the first electrode 100a and the second electrode 100com should be formed of a material which is not corroded when in contact with the electrolyte solution E. In addition, an electrical double layer is formed on a surface of each of the first electrode 100a and the second electrode 100com which come into contact with the electrolyte solution E. Accordingly, both the first electrode 100a and the second electrode 100com are formed of a material configured to form the electrical double layer when in contact with the electrolyte solution E.

As one embodiment, both the first electrode 100a and the second electrode 100com are formed of gold (Au). As another embodiment, a first electrode 100a and a second electrode 100com may be formed of a metal including any one among silver (Ag), mercury (Hg), platinum (Pt), and silver chloride (AgCl).

The probes P may be a material specifically coupled to the target T to be detected using the bio-sensor. In one embodiment, when the target T is deoxyribonucleic acid (DNA) having a specific base sequence, the probes P include a material having a sequence which complementarily binds to the base sequence of the target. Similarly, when DNA, ribonucleic acid (RNA), a protein, a hormone, an antigen, or the like is desired to be detected, a material specifically bound to the DNA, the RNA, the protein, the hormone, the antigen, or the like is used in the probes P.

The probes P are not patterned on the substrate sub and the first and second electrodes 100a and 100com formed on one surface of the substrate, but are randomly disposed. As one embodiment, the probes P may be immobilized and formed on the substrate. For example, an immobilization process may be performed by applying a solution including the probes P to a surface of the substrate, incubating the substrate, and washing the solution. As another example, the immobilization process may be performed by immersing the substrate sub in a solution including the probes P, and removing the solution through evaporation. As another example, the probes P may be disposed by being sprayed, and as another example, the probes P may be randomly disposed through a printing process such as an inkjet printing process using a nozzle and a roll to roll printing process. Accordingly, since a patterning process for selectively disposing the probes P is not required, unlike a related art, material properties of the probes P are not degraded. Accordingly, detection properties of the bio-sensor may be improved.

In the embodiment in which the first electrode 100a and the second electrode 100com are formed of gold (Au), as ends of the probes are processed with a thiol group, adhesion and immobilization between the probes P and the first and second electrodes 100a and 100com may be improved.

A stimulating source DRV is connected to the second electrode 100com and electrically stimulates the second electrode 100com. Readout circuitry RD is connected to the first electrode 100a and receives a detection signal, which is changed according to whether the probes P are coupled to the targets T, from the first electrode 100a. In the illustrated embodiment, the stimulating source DRV configured to electrically stimulate the second electrode 100com is connected to the second electrode 100com, and the readout circuitry RD is connected to the first electrode 100a. However, in another embodiment which is not illustrated, a stimulating source DRV may be connected to a first electrode 100a, and readout circuitry RD may be electrically connected to a second electrode 100com.

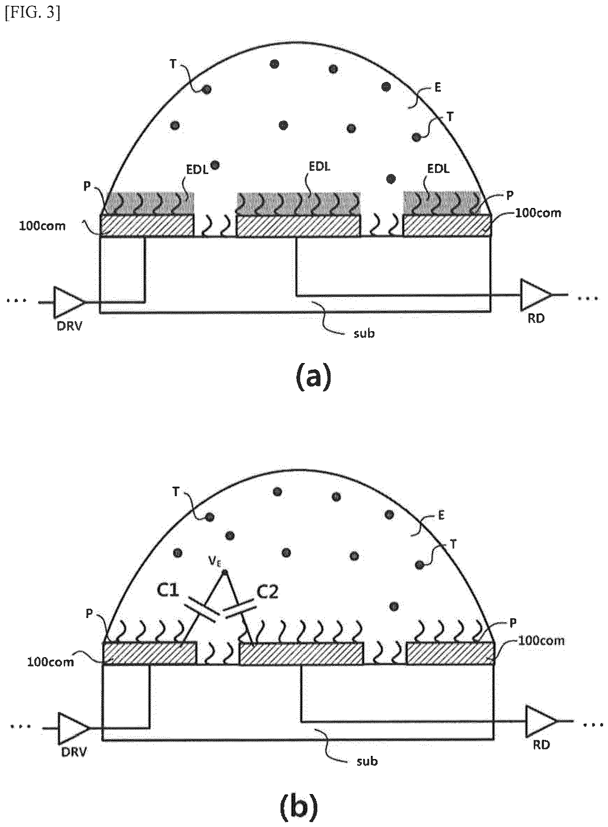

Hereinafter, operation of the bio-sensor having the above-described structure will be described. Continuously referring to FIG. 3A, the electrolyte solution E including the targets T to be detected by the bio-sensor is placed on the bio-sensor. Negative and positive ions in the electrolyte solution E are dissociated, and when the electrolyte solution E comes into contact with the first electrode 100a and the second electrode 100com, as illustrated in FIG. 3A, the negative and positive ions are disposed on surfaces of the first electrode 100a and the second electrode 100com in a layered shape and form an electrical double layer EDL.

When the electrical double layer EDL is formed, the electrolyte E functions as one electrode of a capacitor, the first electrode 100a or the second electrode 100com functions as the other electrode of the capacitor, and the electrical double layer EDL functions as a dielectric material of the capacitor. Capacitance C of the capacitor formed as described above may be calculated by Equation 1.

[Equation 1]

$$C = \varepsilon \frac{A}{d}$$

A separation distance d between the electrodes of the capacitor is a distance between the electrolyte solution E and the first electrode 100a or the second electrode 100com, and corresponds to several angstroms (Å) to several tens of angstroms (Å), which is a thickness of the electrical double layer EDL, because the electrical double layer EDL is interposed between the electrodes and the electrolyte solution.

In addition, when it is assumed that the thicknesses of electrical double layers EDL formed on the surfaces of the first electrode 100a and the second electrode 100com are the same, capacitance C1 generated between the first electrode 100a and the electrolyte solution E and capacitance C2 generated between the second electrode 100com and the electrolyte solution E correspond to surface areas of the first electrode 100a and the second electrode 100com. As one embodiment, when the surface area of the second electrode 100com is ten times greater than that of the first electrode 100a, a value of the capacitance C2 is calculated to be ten times that of the capacitance C1.

This is electrically illustrated in FIG. 3B. When the stimulating source DRV sends an electrical signal corresponding to a voltage $V_{drv}$ to the second electrode 100com, an electric potential $V_E$ of the electrolyte solution E may be calculated by the following Equation 2.

[Equation 2]

$$V_E = V_{drv} \frac{C1}{C1 + C2}$$

That is, the electric potential $V_E$ of the electrolyte solution E has a value corresponding to the capacitance C1 of a capacitor formed at the first electrode 100a and the capacitance C2 of a capacitor formed at the second electrode 100com. The capacitances of the capacitors are proportional to areas of the electrodes in contact with the electrolyte, as seen in Equation 1. Accordingly, when the surface area of the first electrode 100com in contact with the electrolyte solution E is very small in comparison to that of the second electrode 100com in contact with the electrolyte solution E, the corresponding values of the capacitances have the same relation as the surface areas, and thus Equation 2 may approximate the following Equation 3.

[Equation 3]

$$V_E = V_{drv} \frac{\dfrac{C1}{C1}}{\dfrac{C1}{C1} + \dfrac{C2}{C1}} \approx V_{drv}$$

That is, when the surface area of the first electrode 100a is very small in comparison to that of the second electrode 100com and electrical stimulation is provided through the second electrode, the electric potential $V_E$ of the electrolyte may be seen as approximating the electric potential $V_{drv}$ of an electrical signal provided by the stimulating source.

Furthermore, when the bio-sensor according to the embodiment is formed by forming the second electrode 100com, which is a common electrode, to cover an area of a large die through a semiconductor process, and forming the first electrode 100a to have a very small size, a very small difference between the electric potential $V_E$ of the electrolyte solution E and the voltage $V_{drv}$ provided by the stimulating source DRV is maintained.

As one embodiment, in addition, when the surface area of the first electrode 100a is 1/20 of that of the second electrode 100com, the electric potential $V_E$ of the electrolyte solution E is calculated as 0.95 $V_{drv}$ through Equation 3. In another embodiment, when a surface area of a first electrode 100a is 1/10 of that of a second electrode 100com, an electric potential $V_E$ of an electrolyte solution E is calculated as 0.91 $V_{drv}$ through the Equation 3. Accordingly, the bio-sensor according to the embodiment has an advantage in that an electric potential of the electrolyte may be constantly maintained without a voltage drop occurring at a bio-sensor including three electrodes according to the related art.

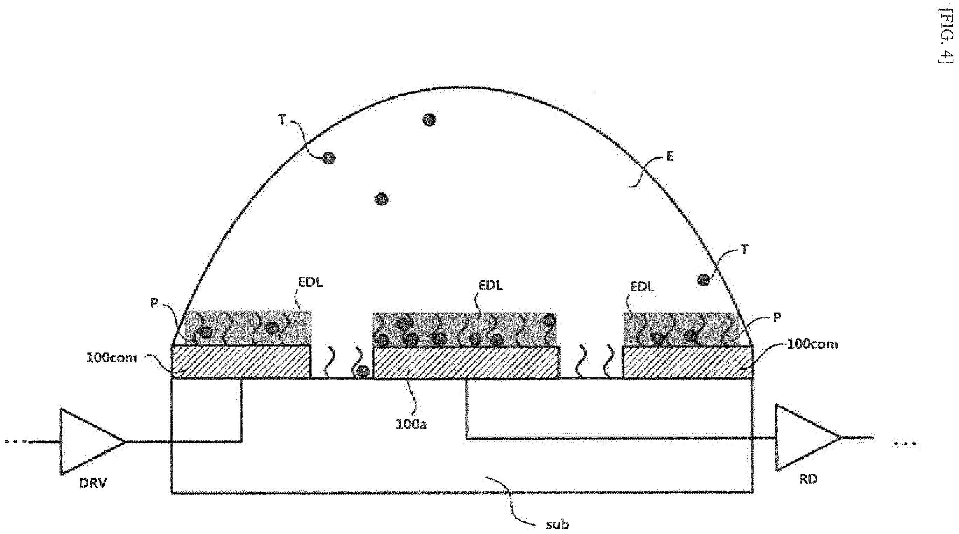

FIG. 4 is a view illustrating an example of a state in which the targets T are coupled to the probes P, and examples in which the targets T are detected will be described with reference to FIG. 4. As one embodiment, since the targets T react with the probes P, a distribution of molecules which cause redox of the surface of the first electrode 100a and/or the second electrode 100com changes, and a change in current may be accordingly detected.

In an embodiment in which the targets T are matrix metalloproteinase 9 (MMP9), which is a cancer metastasis bio-marker, and the probes are methylene blue (MB), which is a peptide having Gly-Pro-Leu-Gly-Met-Trp-Ser-Arg-Cys bonding, when the targets T are not included in the electrolyte solution E, a redox reaction occurs at the electrode due to the MB formed at an end of each of the probes P, and accordingly, a faradaic current is supplied to the electrode.

When the target T is included in the electrolyte solution E, MMP9, which is the target, is coupled to the peptide, which is the probe, and bonding between the Gly and Met of an end of the peptide is broken, and thus the MB is disconnected form the peptide, which is the probe. Accordingly, since the redox reaction occurring due to MB decreases, the faradaic current changes, and thus the presence of the target and/or a concentration of the target may be detected by detecting the change in the faradaic current.

In another embodiment, probes P are coupled to a targets T in an electrical double layer formed by an electrolyte solution E in contact with a first electrode 100a and a second electrode 100com. Although a dielectric layer of a capacitor formed in the first electrode 100a before the probes P are coupled to the targets T is formed as only the electrical double layer, when the probes P are coupled to the targets T, since the electric double layer together with a target material is accommodated in the dielectric layer, a capacitance value of the capacitor formed in the first electrode 100a changes.

When the electric potential $V_E$ of the electrolyte solution E is applied to the capacitor formed at the first electrode 100a, an electrical signal $i_{sense}$ provided by the bio-sensor detecting the target T may be expressed by the following Equation 4.

[Equation 4]

$$i_{sense} = \triangle C \frac{dV_E}{dt}$$

That is, a change in capacitance caused by the targets T coupled to the probes P may change a current value, the readout circuitry RD may detect the change in the current value, a signal may be processed, and thus whether the targets T is included in the electrolyte E or a concentration of the targets may be checked.

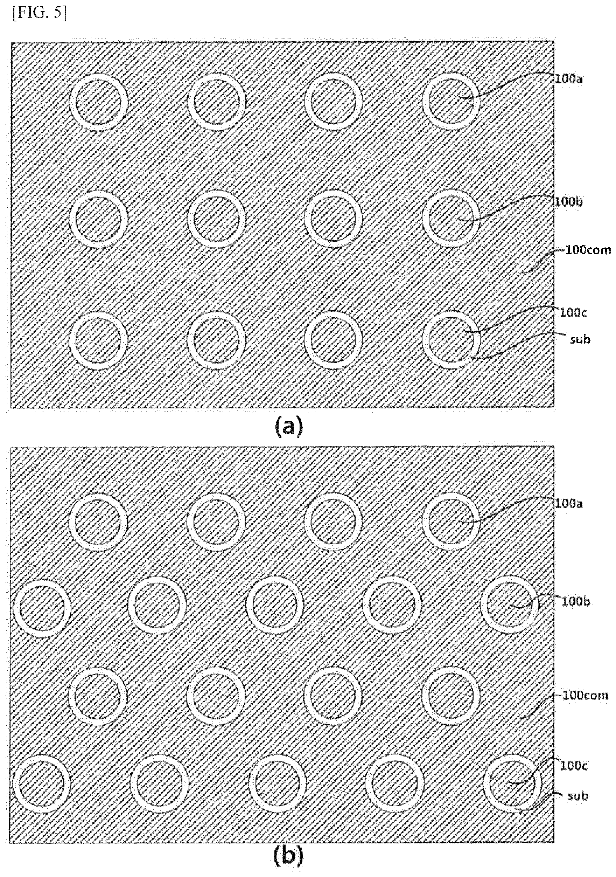

FIG. 5 is a schematic view illustrating the bio-sensor according to the embodiment formed in an array type. Referring to FIG. 5, a bio-sensor array according to the embodiment includes a plurality of island electrodes 100a, 100b, and 100c and a common electrode 100com formed to cover a substrate sub and not configured to be in electrical contact with the island electrodes, and probes P (see FIGS. 1 to 4) are formed on the island electrodes, the substrate, and the common electrode.

FIG. 5A is a view illustrating a bio-sensor array in which the island electrodes 100a, 100b, and 100c are disposed in a rectangular shape, and FIG. 5B is a view illustrating a bio-sensor array in which the island electrodes 100a, 100b, and 100c are diagonally disposed. As described with reference to Equation 3, when a surface area of the common electrode 100com in contact with an electrolyte E (see FIGS. 1 to 4) is greater than those of the island electrodes 100a, 100b, and 100c, an electric potential of the electrolyte approximates an electric potential provided by a stimulating source DRV (see FIG. 2). Therefore, according to the embodiment, the surface area of the common electrode 100com may increase in comparison to those of the island electrodes 100a, 100b, and 100c, and thus there is an advantage in that an electric potential $V_E$ of the electrolyte configured to come into contact with the common electrode 100com and the island electrodes 100a, 100b, and 100c may be maintained to approximate a voltage $V_{drv}$ of an electrical signal provided by the stimulating source.

Furthermore, according to the embodiments illustrated in FIGS. 5A and 5B, since detecting a target object may be simultaneously preformed by the island electrodes 100a, 100b, and 100c configured to form the bio-sensor formed, there is an advantage in that accuracy and sensitivity of the detection of the target materials may be improved.

While the present invention has been disclosed with reference to the embodiments illustrated in the accompanying drawings to facilitate understanding the present invention, it should be understood by those skilled in the art that the embodiments are only examples for implementing the present invention, and various modifications and equivalent other embodiments may be made. Therefore, the true technical protection scope of the present invention should be defined by the appended claims.

[Industrial Applicability]

Industrial applicability has been described above.

## Claims

1. A bio-sensor configured to detect a target, the bio-sensor comprising:

   a substrate;
   a first electrode and a second electrode disposed on the substrate and not electrically connected to each other; and
   probes disposed on the substrate, the first electrode, and the second electrode and coupled to the target.

2. The bio-sensor of claim 1, wherein each of the probes includes a material specifically coupled to the target.

3. The bio-sensor of claim 1, wherein the probes are randomly disposed on the substrate, the first electrode, and the second electrode.

4. The bio-sensor of claim 1, wherein the probes are disposed on the substrate, the first electrode, and the second electrode by being immobilized or sprayed.

5. The bio-sensor of claim 1, wherein the target is included in an electrolyte solution and supplied to the bio-sensor.

6. The bio-sensor of claim 1, wherein the first electrode and the second electrode are formed of gold (Au).

7. The bio-sensor of claim 1, wherein the first electrode and the second electrode include a metal electrode including silver, mercury, platinum, or silver chloride (AgCl).

8. The bio-sensor of claim 1, wherein:

   one of the first electrode and the second electrode is electrically connected to a stimulating source configured to provide electrical stimulation; and
   the other thereof is electrically connected to readout circuitry configured to read a detection signal changed according to whether the probe is coupled to the target.

9. A bio-sensor configured to detect a target which is a biomaterial, the bio-sensor comprising:

   a substrate;
   a first electrode and a second electrode disposed on the substrate, not electrically connected to each other, and having different surface areas; and
   probes disposed on the substrate, the detection electrode, and the common electrode and coupled to the target.

10. The bio-sensor of claim 9, wherein the target is included in an electrolyte solution and provided to the bio-sensor through the electrolyte solution.

11. The bio-sensor of claim 9, wherein a surface area of any one of the first electrode and the second electrode is ten times or more greater than that of the other thereof.

12. The bio-sensor of claim 9, wherein:

    the first electrode is an island type electrode; and
    the second electrode is a common electrode configured to surround the first electrode.

13. The bio-sensor of claim 9, wherein each of the probes incudes a material specifically coupled to the target.

14. The bio-sensor of claim 9, wherein the probes are randomly disposed on the substrate, the first electrode, and the second electrode.

15. The bio-sensor of claim 9, wherein the electrode is formed of gold (Au).

16. The bio-sensor of claim 9, wherein the electrodes include a metal electrode including silver, mercury, platinum, or silver chloride (AgCl).

17. The bio-sensor of claim 9, wherein:

    one of the first electrode and the second electrode is electrically connected to a stimulating source configured to provide electrical stimulation; and
    the other thereof is electrically connected to readout circuitry configured to read a detection signal changed according to whether the probe is coupled to the target.

18. A bio-sensor array in which bio-sensors configured to detect a target, which is a biomaterial, are disposed, the bio-sensor array comprising:

    a substrate;
    a plurality of island electrodes disposed on the substrate;
    a common electrode configured to surround the plurality of island electrodes disposed on the substrate and not in electrical contact with the plurality of island electrodes; and
    probes randomly disposed on the substrate, the plurality of island electrodes, and the common electrode and specifically coupled to the target,

wherein, surface areas of the plurality of island electrodes are smaller than that of the common electrode.

19. The bio-sensor array of claim 18, wherein the probes are sprayed and disposed on the substrate, the plurality of island electrodes, and the common electrode.

[FIG. 1]

[FIG. 2]

[FIG. 3]

(a)

(b)

[FIG. 4]

[FIG. 5]

(a)

(b)

## INTERNATIONAL SEARCH REPORT

| International application No. |
|---|
| **PCT/KR2016/006620** |

**A. CLASSIFICATION OF SUBJECT MATTER**

*G01N 27/327(2006.01)i, G01N 33/53(2006.01)i*

According to International Patent Classification (IPC) or to both national classification and IPC

**B. FIELDS SEARCHED**

Minimum documentation searched (classification system followed by classification symbols)
G01N 27/327; G01N 27/00; G01N 27/30; G01N 33/48; G01N 33/543; G01N 33/53; C12Q 1/68

Documentation searched other than minimum documentation to the extent that such documents are included in the fields searched
Korean Utility models and applications for Utility models: IPC as above
Japanese Utility models and applications for Utility models: IPC as above

Electronic data base consulted during the international search (name of data base and, where practicable, search terms used)
eKOMPASS (KIPO internal) & Keywords: probe, island electrode, common electrode, surface area, bio sensor, target and substrate

**C. DOCUMENTS CONSIDERED TO BE RELEVANT**

| Category* | Citation of document, with indication, where appropriate, of the relevant passages | Relevant to claim No. |
|---|---|---|
| Y | KR 10-2013-0030003 A (SEOUL NATIONAL UNIVERSITY R&DB FOUNDATION) 26 March 2013 See abstract, paragraphs [0021]-[0039], claims 1-10 and figures 1-3. | 1-19 |
| Y | KR 10-2014-0015104 A (SEOUL NATIONAL UNIVERSITY R&DB FOUNDATION) 06 February 2014 See abstract, paragraphs [0043]-[0053], claims 1-9, 42 and figures 2a, 8. | 1-19 |
| A | KR 10-2008-0069995 A (SEOUL NATIONAL UNIVERSITY R&DB FOUNDATION) 29 July 2008 See abstract, claims 1-6 and figures 1-3. | 1-19 |
| A | US 2014-0274760 A1 (FOMINA et al.) 18 September 2014 See abstract, claims 1-8 and figures 1-3. | 1-19 |
| A | KR 10-2011-0133093 A (KOREA ADVANCED INSTITUTE OF SCIENCE AND TECHNOLOGY) 12 December 2011 See abstract, claims 1-6 and figure 2. | 1-19 |

☐ Further documents are listed in the continuation of Box C.　　☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 07 OCTOBER 2016 (07.10.2016) | **11 OCTOBER 2016 (11.10.2016)** |

| Name and mailing address of the ISA/KR | Authorized officer |
|---|---|
| Korean Intellectual Property Office Government Complex-Daejeon, 189 Seonsa-ro, Daejeon 302-701, Republic of Korea | |
| Facsimile No. 82-42-472-7140 | Telephone No. |

Form PCT/ISA/210 (second sheet) (January 2015)

INTERNATIONAL SEARCH REPORT
Information on patent family members

International application No.

**PCT/KR2016/006620**

| Patent document cited in search report | Publication date | Patent family member | Publication date |
|---|---|---|---|
| KR 10-2013-0030003 A | 26/03/2013 | NONE | |
| KR 10-2014-0015104 A | 06/02/2014 | US 2014-0027314 A1 | 30/01/2014 |
| KR 10-2008-0069995 A | 29/07/2008 | CN 101501481 A | 05/08/2009 |
| | | CN 101501481 B | 13/11/2013 |
| | | EP 2054716 A1 | 06/05/2009 |
| | | EP 2054716 A4 | 02/03/2011 |
| | | EP 2054716 B1 | 26/02/2014 |
| | | JP 2010-500558 A | 07/01/2010 |
| | | KR 10-0993167 B1 | 09/11/2010 |
| | | TW 200809190 A | 16/02/2008 |
| | | US 2010-0109645 A1 | 06/05/2010 |
| | | US 8072226 B2 | 06/12/2011 |
| | | WO 2008-018726 A1 | 14/02/2008 |
| US 2014-0274760 A1 | 18/09/2014 | CN 105247359 A | 13/01/2016 |
| | | EP 2972276 A1 | 20/01/2016 |
| | | JP 2016-512335 A | 25/04/2016 |
| | | WO 2014-151690 A1 | 25/09/2014 |
| KR 10-2011-0133093 A | 12/12/2011 | KR 10-1181373 B1 | 19/09/2012 |

Form PCT/ISA/210 (patent family annex) (January 2015)